# FASCICULE DE BREVET EUROPEEN

(11) **EP 2 444 032 B1**
(45) Date de publication et mention de la délivrance du brevet: **30.03.2016**
(21) Numéro de dépôt: 11185537.5
(22) Date de dépôt: 18.10.2011
(51) Int. Cl.: A61F 2/40, A61F 2/30

(54) **Jeu de composants glénoïdiens d'une prothèse d'épaule**
Satz von Glenoidalkomponenten für eine Schulterprothese
Set of glenoidal components of a shoulder prosthesis

(30) Priorité: 22.10.2010 FR 1058647
(43) Date de publication de la demande: 25.04.2012
(73) Titulaire: Tornier, 38330 Montbonnot-Saint-Martin (FR)
(72) Inventeur: Walch, Gilles, 69003 Lyon (FR); Boileau, Pascal, 06200 Nice (FR); Levigné, Christophe, 69300 Caluire (FR); Ferrand, Lucile, 38330 Montbonnot (FR); Deransart, Pierric, 38410 SAINT MARTIN D'URIAGE (FR)
(74) Mandataire: Grand, Guillaume

(56) Documents cités:
- EP-A1- 1 607 067
- EP-A1- 1 782 765
- FR-A1- 2 848 099

## Description

La présente invention concerne un jeu de composants glénoïdiens d'une prothèse d'épaule.

Typiquement, ce genre de composants comporte un corps peu épais, dont les deux faces principales opposées sont adaptées respectivement pour coopérer de manière articulaire avec une tête humérale, soit naturelle, soit prothétique, et pour être appuyée contre la glène d'une omoplate afin d'y être immobilisée. A cette fin, la première face précitée inclut une surface articulaire, généralement sphérique, tandis que la seconde face inclut une surface d'appui, elle aussi sphérique ou bien plane, dans la région centrale de laquelle, souvent, s'étend en saillie au moins un élément d'ancrage osseux dans la glène, telle qu'une quille ou des pions.

Il est connu de prévoir de tels composants glénoïdiens en plusieurs tailles, respectivement définies par les dimensions de la surface articulaire du corps des composants, tandis que les surfaces d'appui sont toutes identiques entre elles dans chaque taille. Un exemple d'un tel jeu de composants glénoïdiens à plusieurs tailles est fourni par FR-A-2 848 099 sur lequel est basé le préambule de la revendication 1.

Ainsi, notamment en fonction de la morphologie du patient, le chirurgien a la possibilité de choisir le composant glénoïdien dont la taille lui semble la plus adaptée au patient opéré. Il en résulte que les performances articulaires de l'épaule prothésée du patient sont très souvent satisfaisantes. Dans le même temps, on constate cependant que, en service, le composant glénoïdien implanté tend progressivement à se desceller de la glène, par usure ou altération mécanique de l'interface entre le corps et la glène.

Le but de la présente invention est de proposer un jeu d'implants glénoïdiens, qui permet au chirurgien d'améliorer la pérennité de la coopération mécanique entre le composant glénoïdien implanté et la glène du patient opéré.

A cet effet, l'invention a pour objet un jeu de composants glénoïdiens d'une prothèse d'épaule, tel que défini à la revendication 1.

Une des idées à la base de l'invention est de mettre à la disposition du chirurgien différentes tailles de composants glénoïdiens, pour chacune desquelles plusieurs composants glénoïdiens sont disponibles avec des surfaces d'appui respectives prévues différentes les unes des autres. De la sorte, en vue de prothéser l'épaule d'un patient donné, le chirurgien décide de la taille du composant glénoïdien qu'il va implanter en fonction de considérations liées à l'articulation à rétablir avec la tête humérale, prothétique ou naturelle, du patient, puis, au sein de la taille que le chirurgien a retenue, le chirurgien choisit le composant glénoïdien dont la surface d'appui est la plus adaptée à la glène du patient, dans son état naturel effectif ou après l'avoir préparée chirurgicalement, de préférence en conservant le plus possible de matière osseuse effectivement présente. Ainsi, l'invention est en rupture avec le « dogme » traditionnel selon lequel le chirurgien doit nécessairement réaliser des opérations de préparation osseuse de la glène du patient assez conséquentes dans le sens où le chirurgien est obligé de retirer une quantité significative de matière osseuse de l'omoplate, pour conformer dans l'omoplate du patient une cavité glénoïdienne adaptée à la géométrie unique et imposée de la surface d'appui glénoïdien du composant que le chirurgien a choisi par sa taille, c'est-à-dire en fonction de sa surface articulaire. A l'inverse, tout en laissant au chirurgien le choix d'une taille pour le composant glénoïdien à implanter, l'invention permet au chirurgien de tenir compte de l'état effectif de la glène opérée. D'ailleurs, contrairement à ce qu'on pourrait penser, l'état osseux de la glène des patients varie dans des proportions non négligeables, et ce de manière décorélée à la coopération articulaire à rétablir grâce à une prothèse d'épaule.

Selon l'invention, les différences entre les surfaces d'appui des différents composants glénoïdiens d'une même taille peuvent tenir à la forme géométrique globale de ces surfaces, ou bien, pour une forme géométrique donnée, aux dimensions de cette forme géométrique. Cet aspect de l'invention sera décrit plus en détail par la suite, à l'aide d'exemples.

Bien entendu, le nombre de tailles présentes dans le jeu selon l'invention, ainsi que, au sein d'une même taille, le nombre de composants glénoïdiens ayant des surfaces d'appui respectives différentes, peuvent être aussi grands que l'on souhaite.

Des caractéristiques additionnelles avantageuses du jeu conforme à l'invention, prises isolément ou selon toutes les combinaisons techniquement possibles, sont spécifiées aux revendications dépendantes 2 à 10.

L'invention sera mieux comprise à la lecture de la description qui va suivre, donnée uniquement à titre d'exemple et faite en se référant aux dessins sur lesquels :
- la figure 1 est une vue en perspective d'un composant glénoïdien appartenant à un jeu conforme à l'invention ;
- la figure 2 est une coupe selon le plan II de la figure 1 ; et
- la figure 3 est une représentation schématique de principe d'un jeu de composants glénoïdiens, conforme à l'invention.

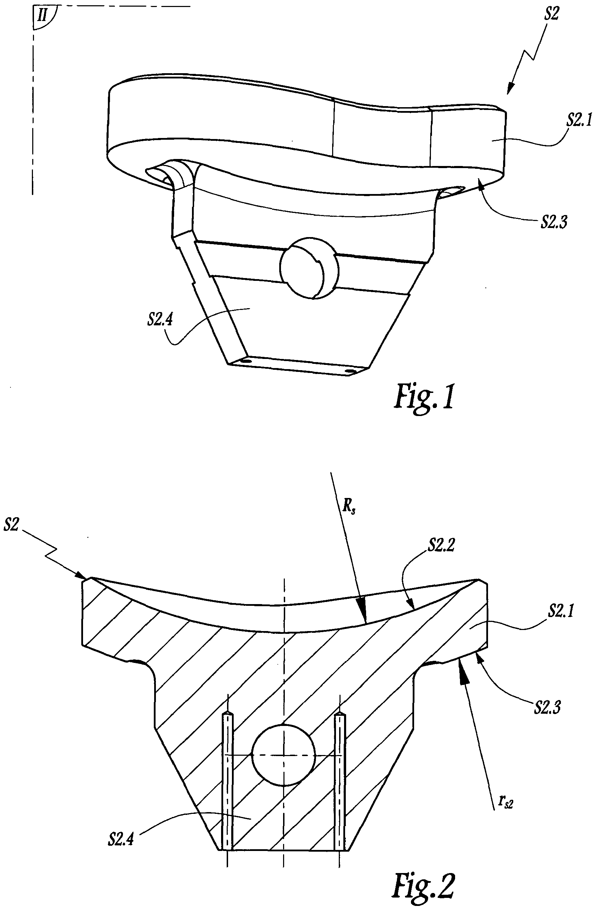

Sur les figures 1 et 2 est représenté un composant prothétique glénoïdien S2.

Ce composant S2 comporte un corps principal S2.1 qui présente des côtes, suivant deux dimensions de l'espace, significativement plus grandes que sa côte suivant sa troisième dimension de l'espace, cette côte correspondant ainsi à l'épaisseur du corps. Le corps S2.1 présente donc deux faces principales opposées, reliées par une face périphérique matérialisant l'épaisseur précitée du corps.

Le corps S2.1 peut aussi bien être monobloc, comme montré sur les figures 1 et 2, que correspondre à l'assemblage fixe de plusieurs parties. De plus, le corps S2.1 est réalisé en un ou des matériaux présentant une rigidité et une tenue appropriée pour, comme expliqué plus en détail ci-après, à la fois coopérer de manière articulaire avec une tête humérale, non représentée, et être solidarisé fixement à la glène osseuse d'une omoplate, également non représentée sur les figures.

L'une des deux faces principales précitées du corps S2.1 délimite une surface articulaire S2.2 conformée pour s'articuler avec la surface sensiblement complémentaire de la tête humérale précitée. En pratique, cette tête humérale est soit naturelle, c'est-à-dire qu'elle correspond à l'épiphyse supérieure naturelle de l'humérus associé à l'omoplate, soit prothétique, c'est-à-dire qu'elle est délimitée par un composant huméral d'une prothèse d'épaule à laquelle appartient le composant glénoïdien S2.

Généralement, la surface articulaire S2.2 du corps S2.1 est sensiblement sphérique. On note R_{S} le rayon de courbure correspondant. Dans l'exemple de réalisation considéré sur les figures 1 et 2, cette surface articulaire S2.2 est concave. A titre de variante non représentée, cette surface articulaire peut être convexe.

En pratique, la surface articulaire S2.2 occupe avantageusement l'essentiel de la face correspondante du corps S2.1, voire la totalité de cette face, comme pour l'exemple illustré sur les figures 1 et 2.

L'autre face principale du corps S2.1 est, quant à elle, adaptée pour être fixée solidairement à la glène précitée. A cette fin, cette face inclut une surface S2.3 d'appui sur la glène, depuis la région centrale de laquelle s'étend en saillie une quille S2.4 d'ancrage dans la glène.

En pratique, en dehors de la quille d'ancrage S2.4, la surface d'appui S2.3 occupe avantageusement l'essentiel de la face correspondante du corps S2.1, voire la totalité de cette face, comme pour l'exemple illustré sur les figures.

A titre de variante non représentée, d'autres éléments d'ancrage osseux que la quille S2.4 peuvent être prévus, en remplacement ou en complément de cette quille. De même, de tels éléments d'ancrage peuvent être totalement absents.

Dans l'exemple de réalisation considéré sur les figures 1 et 2, la surface d'appui S2.3 du corps S2.1 est sensiblement sphérique, ce qui revient à dire qu'elle correspond à une portion de sphère dont on note le rayon de courbure r_{S2}.

En pratique, les surfaces S2.2 et S2.3 peuvent être concentriques ou non. Dans le cas où elles ne sont pas concentriques, leur centre géométrique respectif peuvent être situé ou non dans un même plan médian du corps S2.1.

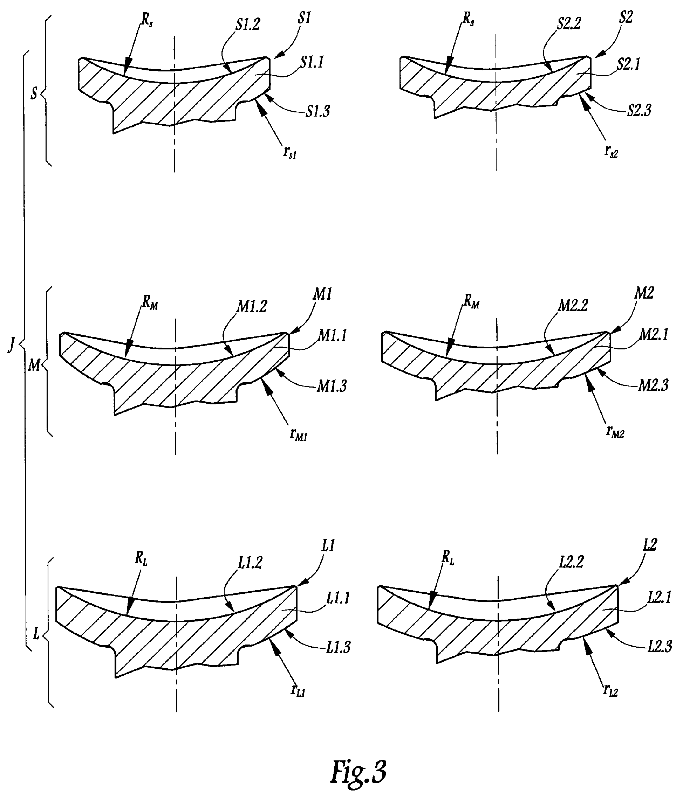

Selon l'invention, le composant glénoïdien S2, qui vient d'être décrit en détail en regard des figures 1 et 2, appartient à un jeu J de six composants glénoïdiens, comme montré sur la figure 3. Ainsi, en plus du composant glénoïdien S2, qui apparaît dans le coin supérieur droit de la figure 3, le jeu J comprend cinq autres composants glénoïdiens, respectivement référencés S1, M1, M2, L1 et L2.

Chacun des composants S1, M1, M2, L1 et L2 comprend un corps S1.1, M1.1, M2.1, L1.1 et L2.1, qui est fonctionnellement similaire au corps S2.1 du composant S2. En particulier, chaque corps S1.1, M1.1, M2.1, L1.1, L2.1 présente, à l'opposé l'une de l'autre, une surface articulaire S1.2, M1.2, M2.2, L1.2 et L.2, fonctionnellement similaire à la surface articulaire S2.2 du composant S2, et une surface d'appui S1.3, M1.3, M2.3, L1.3, L2.3 fonctionnellement similaire à la surface d'appui S2.3 du composant S2.

Le composant S1 présente la même taille que le composant S2, dans le sens où leur surface articulaire respective S1.2 et S2.2 présente sensiblement le même dimensionnement : en particulier, comme pour l'exemple représenté sur la figure 3, ces surfaces articulaires S1.2 et S2.2 sont sensiblement sphériques et présentent le même rayon de courbure R_{S}. Les composants S1 et S2 forment ainsi, au sein du jeu J, un groupe de taille S.

En revanche, le composant S1 se distingue du composant S2 par la géométrie dimensionnelle de sa surface d'appui S1.3 comparativement à celle de la surface d'appui S2.3 du composant S2. Plus précisément, les surfaces d'appui S1.3 et S2.3 présentent toutes deux la même forme en portion de sphère, mais leur rayon de courbure respectif r_{S1}, et r_{S2} sont différents : r_{S1} est strictement inférieur à r_{S2}.

De la même façon, les composants M1 et M2 présentent la même taille et constituent ainsi, au sein du jeu J, un groupe de deux composants, référencé M sur la figure 3. Par analogie à ce qui vient d'être décrit pour les composants S1 et S2, on comprend que les surfaces articulaires M1.2 et M2.2 des composants M1 et M2 présentent sensiblement le même dimensionnement, notamment, dans l'exemple représenté, le même rayon de courbure R_{M}. Le groupe de taille M, contenant les composants M1 et M2, se distingue du groupe S, contenant les composants S1 et S2, par le fait que le rayon R_{M} présente une valeur strictement supérieure à celle du rayon R_{S}.

De plus, comme pour les composants S1 et S2 du groupe de taille S, les surfaces d'appui M1.3 et M2.3 des composants M1 et M2 du groupe de taille M présentent des géométries dimensionnelles différentes, à savoir que ces surfaces d'appui sont toutes deux des portions de sphère dont les rayons respectifs r_{M1} et r_{M2} sont différents, la valeur du rayon r_{M1} étant strictement inférieure à la valeur du rayon r_{M2}.

Dans l'exemple représenté sur la figure 3, on notera que les rayons r_{S1} et r_{M1} ont la même valeur et les rayons r_{S2} et r_{M2} présentent également la même valeur. Autrement dit, lorsqu'on passe du groupe de taille S au groupe de taille M, les rayons de courbure r_{S1} et r_{M1} des surfaces d'appui S1.3 et M1.3 présentent une valeur inchangée. Il en est de même pour les surfaces d'appui S2.3 et M2.3.

Enfin, par transposition des considérations techniques qui précèdent en lien avec les composants S1 et S2 du groupe de taille S et les composants M1 et M2 du groupe de taille M, on comprend que les composants L1 et L2 constituent un groupe de taille L, de sorte que :
- les surfaces articulaires L1.2 et L2.2 des composants L1 et L2 présentent sensiblement le même dimensionnement, notamment le même rayon de courbure L_{L} qui a une valeur strictement supérieure à celle du rayon R_{M}, et
- les surfaces d'appui L1.3 et L2.3 sont toutes deux des portions de sphère présentant des rayons de courbure respectifs r_{L1} et r_{L2}, le rayon r_{L1} présentant une valeur strictement inférieure à celle du rayon r_{L2}.

Dans l'exemple de réalisation représenté sur la figure 3, on notera que le rayon r_{L1} présente une valeur strictement supérieure à celle du rayon r_{M1}, mais inférieure à celle du rayon r_{M2}. Et le rayon r_{L2} présente une valeur strictement supérieure à celle du rayon r_{M2}.

Egalement dans l'exemple de réalisation représenté sur la figure 3, on remarquera que, en plus de présenter des rayons de courbure R_{S}, R_{M} et R_{L} croissants lorsqu'on passe successivement de la taille S à la taille M et à la taille L, les surfaces articulaires des composants glénoïdiens de chacun des groupes de même taille se distinguent par une augmentation de leur étendue bord à bord, notamment dans le plan de coupe de la figure 3. Cette disposition n'est toutefois pas indispensable pour la présente invention.

Ainsi, on comprend que les composants glénoïdiens du jeu J sont ventilés en trois groupes de tailles différentes S, M et L. Selon que le chirurgien choisisse indifféremment l'un des composants S1 et S2 ou bien indifféremment l'un des composants M1 et M2 ou bien indifféremment l'un des composants L1 et L2, le comportement articulaire du composant glénoïdien retenu, vis-à-vis de la tête humérale du patient, est fixé. En revanche, pour chacune des tailles S, M et L, le choix par le chirurgien d'un des deux composants disponibles lui permet de tenir compte de l'état effectif de la glène du patient opéré : notamment, le chirurgien va choisir celui des deux composants disponibles dont la courbure de la surface d'appui est la plus adaptée à la configuration géométrique effective de la glène, en particulier pour limiter l'étendue de la préparation préalable de la glène, en limitant ainsi la perte de substance osseuse, tout en optimisant l'étendue de l'interface d'appui réalisée entre la glène et le composant retenu lors de l'implantation de ce dernier.

En pratique, la différence entre les rayons de courbure des surfaces d'appui des composants d'une même taille est, de préférence, supérieure ou égale à 4 mm, voire supérieure ou égale à 8 mm. Ainsi, un exemple de quantification numérique en lien avec la forme de réalisation du jeu J montré à la figure 3 est le suivant :
- r_{S1} = 34 mm,
- r_{S2} = 38 mm,
- r_{M1} = 34 mm,
- r_{M2} = 38 mm,
- r_{L1} = 36 mm, et
- r_{L2} = 44 mm.

Bien entendu, divers aménagements et variantes sont envisageables pour le jeu de composants glénoïdiens J. A titre d'exemple, au moins l'un des trois groupes de tailles différentes S, M et L peut inclure plus de deux composants glénoïdiens, les surfaces d'appui respectives des au moins trois composants glénoïdiens présents ayant respectivement des géométries dimensionnelles différentes. De même, le jeu peut être prévu qu'avec deux groupes de tailles différentes, par exemple S et M, ou S et L ou M et L. Bien entendu, le jeu peut également inclure plus de trois groupes de tailles différentes.

Suivant une variante non représentée de l'invention, la différence de géométries dimensionnelles entre les surfaces d'appui des composants glénoïdiens d'une même taille ne correspond pas à une différence dimensionnelle d'une même forme géométrique, comme c'est le cas pour le jeu J, mais cette différence repose sur la présence d'autant de formes géométriques différentes que de composants glénoïdiens présents dans le groupe d'une même taille. Plus précisément, au sein de chaque groupe d'une même taille, l'un des composants glénoïdiens présente, par exemple, une surface d'appui qui est plane tandis que l'autre composant glénoïdien présente une surface d'appui qui est incurvée convexe. Plus généralement, les surfaces d'appui respectives'des au moins deux composants glénoïdiens présents dans le groupe précité présentent alors des formes géométriques différentes qui sont choisies parmi, au moins, une surface plane, une surface incurvée convexe, une surface incurvée concave et une surface en gradins.

A titre optionnel, plutôt que d'être rigoureusement lisses comme sur les figures, les surfaces d'appui des composants du jeu J peuvent présenter une rugosité, voire des macro-aspects, tels que des rainures, des picots pyramidaux, des alvéoles coniques, etc., afin de renforcer l'immobilisation du composant à la glène.

## Revendications

1. Jeu (J) de composants glénoïdiens d'une prothèse d'épaule, comprenant des composants glénoïdiens (S1, S2, M1, M2, L1, L2) qui inclut chacun un corps (S1.1, S2.1, M1.1, M2.1, L1.1, L2.1) délimitant, sur respectivement deux de ses faces opposées, une surface articulaire (S1.2, S2.2, M1.2, M2.2, L1.2, L2.2), destinée à coopérer de manière articulaire avec une tête humérale, prothétique ou naturelle, et une surface (S1.3, S2.3, M1.3, M2.3, L1.3, L2.3) d'appui contre la glène d'une omoplate, dans lequel les composants glénoïdiens sont prévus en plusieurs tailles différentes (S, M, L), respectivement définies par les dimensions de la surface articulaire de leur corps, **caractérisé en ce que**, dans chaque taille (S, M, L), il est prévu au moins deux composants glénoïdiens (S1 et S2, M1 et M2, L1 et L2) dont les surfaces d'appui (S1.3 et S2.3, M1.3 et M2.3, L1.3 et L2.3) de leur corps respectif (S1.1 et S2.1, M1.1 et M2.1, L1.1 et L2.1) présentent respectivement des géométries dimensionnelles différentes.

2. Jeu suivant la revendication 1, **caractérisé en ce que** lesdites géométries dimensionnelles, respectivement prévues différentes pour au moins deux des composants glénoïdiens (S1 et S2, M1 et M2, L1 et L2) ayant une même taille (S, M, L), correspondent à une même forme de surface incurvée convexe mais présentent autant de courbures différentes.

3. Jeu suivant la revendication 2, **caractérisé en ce que** ladite même forme de surface incurvée convexe est sensiblement une portion de sphère, de sorte que lesdites géométries dimensionnelles, respectivement prévues différentes pour au moins deux des composants glénoïdiens (S1 et S2, M1 et M2, L1 et L2) ayant une même taille (S, M, L), correspondent à autant de rayons de courbure différents (r_{S1} et r_{S2}, r_{M1} et r_{M2}, r_{L1} et r_{L2}).

4. Jeu suivant la revendication 3, **caractérisé en ce que**, pour certaines tailles (S, M, L) au moins, la différence entre les rayons de courbure respectifs (r_{S1} et r_{S2}, r_{M1} et r_{M2}, r_{L1} et r_{L2}) des surfaces d'appui (S1.3 et S2.3, M1.3 et M2.3, L1.3 et L2.3) desdits au moins deux composants (S1 et S2, M1 et M2, L1 et L2) ayant une même taille (S, M, L) est supérieure à 4 mm, de préférence supérieure à 8 mm.

5. Jeu suivant l'une des revendications 3 ou 4, **caractérisé en ce qu'**au moins l'un desdits rayons de courbure (r_{S1} et r_{S2}, r_{M1} et r_{M2}), respectivement prévus différents pour une même taille (S, M), présente une valeur qui est inchangée au moins une fois lorsque, parmi les tailles, on passe d'une taille inférieure à une taille supérieure.

6. Jeu suivant l'une quelconque des revendications 3 à 5, **caractérisé en ce qu'**au moins l'un desdits rayons de courbure (r_{M1} et r_{M2} et r_{L1} et r_{L2}), respectivement prévus différents pour une même taille (M, L), présente une valeur qui augmente au moins une fois lorsque, parmi les tailles, on passe d'une taille inférieure à une taille supérieure.

7. Jeu suivant la revendication 1, **caractérisé en ce que** lesdites géométries dimensionnelles, respectivement prévues différentes pour au moins deux des composants glénoïdiens ayant une même taille, correspondent à autant de formes géométriques différentes parmi, au moins, une surface plane, une surface incurvée convexe, une surface incurvée concave et une surface en gradins.

8. Jeu suivant l'une quelconque des revendications précédentes, **caractérisé en ce qu'**au moins l'une desdites géométries dimensionnelles, respectivement prévues différentes pour au moins deux des composants glénoïdiens (S1 et S2, M1 et M2) ayant une même taille, se retrouve à l'identique dans au moins deux (S, M) des différentes tailles (S, M, L).

9. Jeu suivant l'une quelconque des revendications précédentes, **caractérisé en ce qu'**au moins un des composants glénoïdiens (S1, S2, M1, M2, L1, L2) du jeu (J) est pourvu d'au moins un élément (S2.4) d'ancrage dans la glène de l'omoplate, qui s'étend en saillie de la surface d'appui (S2.3) de ce composant glénoïdien.

10. Jeu suivant l'une quelconque des revendications précédentes, **caractérisé en ce que** la surface d'appui (S1.3, S2.3, M1.3, M2.3, L1.3, L2.3) de chacun des composants glénoïdiens (S1, S2, M1, M2, L1, L2) du jeu (J) occupe, en dehors du ou des éléments d'ancrage (S2.4) potentiellement présents, l'essentiel, voire la totalité de la face correspondante du corps (S1.1, S2.1, M1.1, M2.1, L1.1, L2.1) du composant glénoïdien.

## Patentansprüche

1. Satz (J) von Gelenkkomponenten einer Schulterprothese, aufweisend Gelenkkomponenten (S1, S2, M1, M2, L1, L2), die jeweils einen Körper (S1.1, S2.1, M1.1, M2.1, L1.1, L2.1) aufweisen, der an jeweils zugeordnet zwei von seinen entgegengesetzten Seiten eine Gelenkfläche (S1.2, S2.2, M1.2, M2.2, L1.2, L2.2), die dazu bestimmt ist, in gelenkiger Weise mit einem prothetischen oder natürlichen Oberarmkopf zusammenzuwirken, und eine Fläche (S1.3, S2.3, M1.3, M2.3, L1.3, L2.3) zur Abstützung gegen die Gelenkpfanne eines Schulterblatts begrenzt, wobei die Gelenkkomponenten in mehreren unterschiedlichen Größen (S, M, L) vorgesehen sind, die jeweilig definiert sind durch die Abmessungen der Gelenkfläche ihres Körpers, **dadurch gekennzeichnet, dass** in jeder Größe (S, M, L) wenigstens zwei Gelenkkomponenten (S1 und S2, M1 und M2, L1 und L2) vorgesehen sind, deren Flächen zur Abstützung (S1.3 und S2.3, M1.3 und M2.3, L1.3 und L2.3) ihres jeweiligen Körpers (S1.1 und S2.1, M1.1 und M2.1, L1.1 und L2.1) jeweils unterschiedliche Abmessungsgeometrien haben.

2. Satz gemäß Anspruch 1, **dadurch gekennzeichnet, dass** die besagten Abmessungsgeometrien, die jeweils unterschiedlich vorgesehen sind für wenigstens zwei Gelenkkomponenten (S1 und S2, M1 und M2, L1 und L2), die eine gleiche Größe (S, M, L) haben, zu einer gleichen konvex gekrümmten Flächen-Form korrespondieren, aber dennoch unterschiedliche Krümmungen haben.

3. Satz gemäß Anspruch 2, **dadurch gekennzeichnet, dass** die besagte gleich konvex gekrümmte Flächen-Form im Wesentlichen ein Sphären-Abschnitt ist, sodass die besagten Abmessungsgeometrien, die jeweils für wenigstens zwei Gelenkkomponenten (S1 und S1, M1 und M2, L1 und L2), welche die gleiche Größe (S, M, L) haben, unterschiedlich vorgesehen sind, zu genauso vielen Krümmungsradien (r_{S1} und r_{S2}, r_{M1} und r_{M2}, r_{L1} und r_{L2}) korrespondieren.

4. Satz gemäß Anspruch 3, **dadurch gekennzeichnet, dass** für wenigstens einige Größen (S, M, L) der Unterschied zwischen den jeweiligen Krümmungsradien (r_{S1} und r_{S2}, r_{M1} und r_{M2}, r_{L1} und r_{L2}) der Flächen zur Abstützung (S1.3 und S2.3, M1.3 und M2.3, L1.3 und L2.3) der besagten wenigstens zwei Komponenten (S1 und S2, M1 und M2, L1 und L2), die eine gleiche Größe (S, M, L) haben, größer als 4mm, bevorzugt größer als 8mm ist.

5. Satz gemäß irgendeinem der Ansprüche 3 oder 4, **dadurch gekennzeichnet, dass** wenigstens einer der besagten Krümmungsradien (r_{S1} und r_{S2}, r_{M1} und r_{M2}), die jeweils unterschiedlich vorgesehen sind für eine gleiche Größe (S, M), einen Wert hat, der wenigstens einmal unverändert bleibt, wenn man, unter den Größen, von einer kleineren Größe zu einer größeren Größe geht.

6. Satz gemäß irgendeinem der Ansprüche 3 bis 5, **dadurch gekennzeichnet, dass** wenigstens einer der besagten Krümmungsradien (r_{M1} und r_{M2} und r_{L1} und r_{L2}), die jeweils unterschiedlich vorgesehen sind für eine gleiche Größe (M, L), einen Wert hat, der wenigstens einmal ansteigt, wenn man, unter den Größen, von einer unteren Größe zu einer oberen Größe geht.

7. Satz gemäß Anspruch 1, **dadurch gekennzeichnet, dass** die besagten Abmessungsgeometrien, die jeweils unterschiedlich vorgesehen sind für wenigstens zwei der Gelenkkomponenten, die eine gleiche Größe haben, zu genauso vielen unterschiedlichen geometrischen Formen korrespondieren unter, zumindest, einer ebenen Fläche, einer konvex gekrümmten Fläche, einer konkav gekrümmten Fläche und stufenförmigen Fläche.

8. Satz gemäß irgendeinem der vorhergehenden Ansprüche, **dadurch gekennzeichnet, dass** wenigstens eine der besagten Abmessungsgeometrien, die jeweils unterschiedlich vorgesehen sind für wenigstens zwei der Gelenkkomponenten (S1 und S1, M1 und M2), die eine gleiche Größe haben, sich identisch bei wenigstens zwei (S, M) der unterschiedlichen Größen (S, M, L) vorfindet.

9. Satz gemäß irgendeinem der vorhergehenden Ansprüche, **dadurch gekennzeichnet, dass** wenigstens eine der Gelenkkomponenten (S1, S2, M1, M2, L1, L2) des Spiels (J) versehen ist mit wenigstens einem Element (S2.4) zur Verankerung in der Gelenkpfanne des Schulterblatts, das sich von der Fläche zur Abstützung (S2.3) dieser Gelenkkomponente aus vorstehend erstreckt.

10. Satz gemäß irgendeinem der vorhergehenden Ansprüche, **dadurch gekennzeichnet, dass** die Fläche zur Abstützung (S1.3, S2.3, M1.3, M2.3, L1.3, L2.3) jeder der Gelenkkomponenten (S1, S2, M1, M2, L1, L2) des Satzes (J), abgesehen von dem oder den potentiell vorliegenden Elementen zur Verankerung (S2.4), den Hauptteil, wenn nicht sogar die Gesamtheit der Seite einnimmt, die zu dem Körper (S1.1, S2.1, M1.1, M2.1, L1.1, L2.1) der Gelenkkomponente korrespondiert.

## Claims

1. A set (J) of glenoid components of a shoulder prosthesis,
comprising glenoid components (S1, S2, M1, M2, L1, L2) that each include a body (S1.1, S2.1, M1.1, M2.1, L1.1, L2.1) defining, on two of its opposite faces, respectively, a joint surface (S1.2, S2.2, M1.2, M2.2, L1.2, L2.2), intended to cooperate hingedly with a humeral head, prosthetic or natural, and a bearing surface (S1.3, S2.3, M1.3, M2.3, L1.3, L2.3) bearing against the glenoid cavity of a scapula,
in which set the glenoid components are provided in several different sizes (S, M, L), respectively defined by the dimensions of the joint surface of their body,
**characterized in that**, in each size (S, M, L), at least two glenoid components (S1 and S2, M1 and M2, L1 and L2) are provided whereof the bearing surfaces (S1.3 and S2.3, M1.3 and M2.3, L1.3 and L2.3) of their respective body (S1.1 and S2.1, M1.1 and M2.1, L1.1 and L2.1) respectively have different dimensional geometries.

2. The set according to claim 1, **characterized in that** said dimensional geometries, respectively provided to be different for at least two of the glenoid components (S1 and S2, M1 and M2, L1 and L2) having a same size (S, M, L), correspond to a same convex curved surface shape but have as many different curves.

3. The set according to claim 2, **characterized in that** said same convex curved surface shape is substantially a sphere portion, so that said dimensional geometries, respectively provided to be different for at least two of the glenoid components (S1 and S2, M1 and M2, L1 and L2) having a same size (S, M, L), correspond to as many different curve radii (r_{S1} and r_{S2}, r_{M1} and r_{M2}, r_{L1} and r_{L2}).

4. The set according to claim 3, **characterized in that**, for at least some sizes (S, M, L), the difference between the respective curve radii (r_{S1} and r_{S2}, r_{M1} and r_{M2}, r_{L1} and r_{L2}) of the bearing surfaces (S1.3 and S2.3, M1.3 and M2.3, L1.3 and L2.3) of said at least two components (S1 and S2, M1 and M2, L1 and L2) having a same size (S, M, L) is greater than 4 mm, preferably greater than 8 mm.

5. The set according to one of claims 3 or 4, **characterized in that** at least one of the curve radii (r_{S1} and r_{S2}, r_{M1} and r_{M2}, r_{L1} and r_{L2}), respectively provided to be different for a same size (S, M), has a value that is unchanged at least once when, among the sizes, one goes from a smaller size to a larger size.

6. The set according to one of claims 3 to 5, **characterized in that** at least one of the curve radii (r_{S1} and r_{S2}, r_{M1} and r_{M2}, r_{L1} and r_{L2}), respectively provided to be different for a same size (M, L), has a value that increases at least once when, among the sizes, one goes from a smaller size to a larger size.

7. The set according to claim 1, **characterized in that** said dimensional geometries, respectively provided to be different for at least two of the glenoid components having a same size, correspond to as many different geometric shapes among, at least, a planar surface, a convex curved surface, a concave curved surface, and a terraced surface.

8. The set according to any one of the preceding claims, **characterized in that** at least one of said dimensional geometries, respectively provided to be different for at least two of the glenoid components (S1 and S2, M1 and M2, L1 and L2) having a same size, is found identically in at least two (S, M) of the different sizes (S, M, L).

9. The set according to any one of the preceding claims, **characterized in that** at least one of the glenoid components (S1, S2, M1, M2, L1, L2) of the set (J) is provided with at least one anchor element (S2.4) in the glenoid cavity of the scapula, which protrudes from the bearing surface (S2.3) of said glenoid component.

10. The set according to any one of the preceding claims, **characterized in that** the bearing surface (S1.3, S2.3, M1.3, M2.3, L1.3, L2.3) of each of the glenoid components (S1, S2, M1, M2, L1, L2) of the set (J) occupies, outside the anchoring element(s) (S2.4) that may be present, most or all of the corresponding face of the body (S1.1, S2.1, M1.1, M2.1, L1.1, L2.1) of the glenoid component.
